# FASCICULE DE BREVET EUROPEEN

(11) **EP 1 960 011 B1**
(45) Date de publication et mention de la délivrance du brevet: **06.04.2016**
(21) Numéro de dépôt: 06841875.5
(22) Date de dépôt: 06.12.2006
(51) Int. Cl.: A61L 27/26, A61L 27/24, A61L 27/60

(54) **UTILISATION D' UN POLYSACCHARIDE EXCRETE PAR L'ESPECE VIBRIO DIABOLICUS A DES FINS D'INGENIERIE DES TISSUS CONJONCTIFS NON MINERALISES**
VERWENDUNG EINES POLYSACCHARIDS, DAS VON DER VIBRIO-DLABOLICUS-SPEZIE AUSGESCHIEDEN WIRD ZUR HERSTELLUNG VON NICHT MINERALISIERTEM VERBINDUNGSGEWEBE
USE OF A POLYSACCHARIDE WHICH IS EXCRETED BY THE VIBRIO DIABOLICUS SPECIES FOR THE ENGINEERING OF NON-MINERALISED CONNECTIVE TISSUE

(30) Priorité: 07.12.2005 FR 0512413
(43) Date de publication de la demande: 27.08.2008
(73) Titulaire: Institut Francais de Recherche pour l'Exploitation de la Mer (IFREMER), 92130 Issy-les-Moulineaux Cedex (FR); Université René Descartes - Paris 5, 75270 Paris Cedex 06 (FR)
(72) Inventeur: SENNI, Karim, F-93600 Aulnay sous Bois (FR); SINQUIN, Corinne, F-44300 Nantes (FR); COLLIEC-JOUAULT, Sylvia, F-44100 Nantes (FR); GODEAU, Gaston-Jacques, F-92160 Antony (FR); GUEZENNEC, Jean, F-29280 Plouzane (FR)
(74) Mandataire: Cabinet Plasseraud
(86) Numéro de dépôt international: PCT/FR2006/002668
(87) Numéro de publication internationale: WO 2007/066009

(56) Documents cités:
- EP-A- 1 384 774
- WO-A-02/02051
- ZANCHETTA P ET AL: "A new bone-healing material: a hyaluronic acid-like bacterial exopolysaccharide." CALCIFIED TISSUE INTERNATIONAL. JAN 2003, vol. 72, no. 1, janvier 2003 (2003-01), pages 74-79, XP002402109 ISSN: 0171-967X

## Description

La présente invention se rapporte à l'ingénierie des tissus conjonctifs non minéralisés, notamment les tissus de recouvrement (la peau, la gencive le cartilage, les tendons).

Des bactéries productrices d'exopolysaccharide (EPS) ont été isolées parmi des microorganismes provenant des écosystèmes hydrothermaux profonds. HE800 est un EPS produit par la souche *Vibrio diabolicus.* Sa masse molaire moyenne en poids est d'environ 800 000 g/mol à l'état natif. Il est caractérisé par une séquence osidique répétitive linéaire originale constituée par 4 résidus osidiques :
**[(-3)-DGlcNacβ(1-4)DGlcAβ(1-4)DGlcAβ(1-4)DGalNacα(1-)]n**

HE800 a été décrit dans la demande internationale au nom de IFREMER publiée sous le numéro WO9838327 ainsi que dans les articles suivants : Raguénès et al, Int J Syst Bact, 1997, 47, 989-995 et Rougeaux et al, Carbohyd. Res, 1999, 322, 40-45. De nombreuses applications pour cet exopolysaccharide ont été décrites. A titre d'exemple d'application, on peut citer la demande internationale WO0202051, qui décrit les propriétés bénéfiques de HE800 en cicatrisation osseuse. Aucune application de HE800 n'est à ce jour connue en ce qui concerne l'ingénierie des tissus conjonctifs non minéralisés.

Le tissu conjonctif se caractérise par la présence entre ses cellules d'une très abondante matrice extracellulaire.
La matrice extracellulaire constitue l'armature du tissu conjonctif non minéralisé. Elle confère aux tissus conjonctifs non minéralisés leur forme, leur résistance mécanique, leur souplesse et assure des fonctions physiologiques importantes. L'organisation de la trame collagénique est un élément essentiel de la structuration tissulaire. En effet, les collagènes et notamment les collagènes fibrillaires constituent la classe protéique majoritaire des matrices extracellulaires et en particulier celles du derme de la gencive et du cartilage.

La matrice extracellulaire est également nécessaire au maintien de l'état différencié des cellules qui la synthétisent et la remodèlent, en particulier les cellules mésenchymateuses (fibroblastes, myofibroblastes, chondroncytes, péricytes...) qui sont les cellules reines des tissus conjonctifs non minéralisés. On distingue notamment les myofibroblastes qui prolifèrent lors de la cicatrisation mais persistent lors de processus inflammatoires chroniques aboutissant à l'installation de fibroses.

Le but principal de l'ingénierie tissulaire est la reconstruction tant des tissus que des organes humains. Plusieurs voies sont dévéloppées.
Une première voie consiste à implanter une structure de guidage au sein d'un tissu endommagé. La structure de guidage sert de moule au tissu à reconstruire. La structure peut être éventuellement enrichie avec des molécules stimulant la croissance cellulaire. A titre d'exemple de cette approche d'ingénierie tissulaire on peut citer EP1555035, qui décrit un implant bioabsorbable constitué d'une matrice mixte collagene-glycosaminoglycanne pontée. Cette matrice constitue une structure de guidage qui a pour objet de baliser le potentiel régénératif du tissu.

Une deuxième voie d'ingénierie tissulaire consiste à reconstruire *ex vivo* des substituts de tissus à partir de cellules vivantes pour des usages *in vivo* ou *ex vivo.* L'objectif est de reproduire l'architecture tissulaire. La méthode basée sur la production d'un gel de collagène a été la première à démontrer les vastes possibilités du génie tissulaire. Les premiers travaux de reconstruction tissulaire réalisés ont mis en évidence que l'incorporation de fibroblastes dans un gel de collagène permettait de produire des équivalents de derme pouvant être ensuite épidermisés par ensemencement de kératinocytes à leur surface. Le résultat final, une peau reconstruite vivante, peut atteindre un excellent niveau de différenciation dans des conditions de culture adéquates et de nombreux aspects de la différenciation terminale des kératinocytes peuvent ainsi être reproduits. Cette méthode de reconstruction tissulaire s'est avérée applicable pour l'élaboration de nombreux autres organes à des visées cliniques (transplantation) ou fondamentales (modélisation tissulaire in vitro). Cependant, il n'est pas certain que cette méthode permette d'obtenir des tissus ayant la résistance mécanique indispensable à leur application clinique.

Cet aspect mécanique est l'une des raisons qui ont poussé au développement d'une seconde méthode, basée sur l'utilisation de biomatériaux colonisables par les cellules et leur culture *in vitro.* Ainsi, les cellules une fois incorporées dans ces supports sécrètent des quantités variées de matrice extracellulaire conduisant à la reconstruction d'une structure tissulaire proche du tissu d'origine, mais comprenant également la trame biodégradable du biomatériau. A titre d'exemple de procédé d'ingénierie tissulaire on peut citer WO03041568 qui décrit une matrice tridimensionnelle comprenant une matrice de fibrine et des fibroblastes. Cette matrice permet de générer des équivalents de tissus qui peuvent être greffés.
Un des objets de la présente invention est une matrice aux propriétés mécaniques améliorées qui favorise la prolifération des fibroblastes.

Les inventeurs ont mis en évidence, de façon surprenante et inattendue, qu'un polysaccharide de masse molaire moyenne en poids comprise entre 500 000 et 2 000 000 g/mol, caractérisé par une séquence osidique répétitive linéaire comprenant les 4 résidus osidiques suivants :
**[(-3)-DGlcNacβ(1-4)DGlcAβ(1-4)DGlcAβ(1-4)DGalNacα(1-)]** présente les propriétés suivantes : il induit une sélection des souches fibroblastiques, il stimule la mobilisation et la prolifération des fibroblastes dans la matrice extracellulaire, il accélère la fibrillation collagénique et favorise ainsi la reconstitution de la matrice extracellulaire.
Ce polysaccharide permet la restructuration de la trame collagénique des tissus conjonctifs non minéralisés, et il constitue un support permettant l'adhésion et la prolifération cellulaire des fibroblastes.

Ainsi, de par ses propriétés, le polysaccharide permet la fabrication de matrice de collagène fibrillaire aux propriétés améliorées. La trame collagénique des matrices de collagènes fibrillaires comprenant le polysaccharide présente une meilleure résistance devant des facteurs physiques tels que la température et les contraintes mécaniques. Enfin, il favorise la culture des cellules mésenchymateuses en particulier la culture des fibroblastes et permet la réalisation de substitut de tissus.

L'invention a pour objet l'utilisation d'un polysaccharide ou un sel de ce polysaccharide de masse molaire moyenne en poids comprise entre 500 000 et 2 000 000 g/mol, préférentiellement comprise entre 700 000 et 900 000 g/mol, caractérisé par une séquence osidique répétitive linéaire comprenant les 4 résidus osidiques suivants :
**[(-3)-DGlcNacβ(1-4)DGlcAβ(1-4)DGlcAβ(1-4)DGalNacα(1-)]** à des fins d'ingénierie des tissus conjonctifs non minéralisés.

La présente invention a pour objet une matrice de collagène comprenant un polysaccharide ou un sel de ce polysaccharide de masse molaire moyenne en poids comprise entre 500 000 et 2 000 000 g/mol, préférentiellement comprise entre 700 000 et 900 000 g/mol, caractérisé par une séquence osidique répétitive linéaire comprenant les 4 résidus osidiques suivants :
**[(-3)-DGlcNacβ(1-4)DGlcAβ(1-4)DGlcAβ(1-4)DGalNacα(1-)]**

Typiquement le polysaccharide peut se présenter sous la forme d'un sel.
Typiquement le polysaccharide est un polysaccharide excrété par l'espèce *Vibrio diabolicus* de taille comprise entre 500 000 et 2 000 000 daltons. Des modes de préparation ont été décrits dans les documents suivantes : WO 98/38327, Raguénès et al, Int J Syst Bact, 1997, 47, 989-995 et Rougeaux et al, Carbohyd. Res, 1999, 322, 40-45.

Typiquement le collagène de la matrice est un collagène choisi dans le groupe constitué des collagènes fibrillaires tels que les collagènes de type I, II, III, V et XI ou d'un mélange de ceux-ci. Préférentiellement le collagène est un collagène de type I.
Typiquement pour fabriquer une telle matrice de collagène, l'homme du métier utilisera les techniques couramment utilisées pour la fabrication des matrices de collagènes à partir de collagènes fibrillaires acido-solubles. En présence du polysaccharide selon l'invention, les collagènes fibrillaires acido-solubles se fibrillent naturellement après neutralisation du pH. Alternativement la matrice de collagène selon l'invention pourra être obtenue par pontage du polysaccharide selon l'invention avec le collagène. Pour réaliser le pontage l'homme du métier utilisera les techniques couramment utilisées pour le pontage de polysaccharides avec le collagène. EP1374857 est une illustration d'une technique de pontage utilisable.

La présente invention a également pour objet une matrice comprenant le polysaccharide tel que décrit précédemment caractérisée en ce que le polysaccharide a été rendu insoluble par réticulation à l'aide d'un ou plusieurs agents réticulants.
Typiquement pour réticuler afin de rendre insoluble le polysaccharide selon l'invention, l'homme du métier utilisera les techniques couramment utilisées pour la réticulation des polysaccharides. A titre d'exemples d'agents réticulants, on peut citer le trimétaphosphate de sodium, l'épichlorhydrine, la divinylsulfone, le glutaraldéhyde et les bisépoxyranes comme par exemple l'éther de 1,4-butanediol-bis(époxypropyle) et l'éther de 1,4-butanediol-diglycidyle.

De manière avantageuse, les matrices selon l'invention pourront comprendre en outre un facteur de croissance qui favorise la colonisation de la matrice par les cellules mésenchymateuses, en particulier par les fibroblastes.

Préférentiellement le facteur de croissance pourra être choisi dans le groupe consistant en TGF-beta, PDGF, FGFs, BMPs (bone morphogenetic proteins), VEGF, CTGF (connective tissue growth factor).

Typiquement les matrices selon l'invention pourront servir de dispositif médical, résorbable ou non, ou d'implant ou pourront être intégrées dans un dispositif médical ou dans un implant. De telles matrices permettront le remplacement mécanique et fonctionnel de structures endommagées avec un minimum de réactions indésirables. Ces matrices une fois déposées sur le tissu ou implantée dans un tissu endommagé serviront de structure de guidage et balisera le potentiel régénératif du tissu. La présence du polysaccharide au sein de la matrice accélère la régénération en accélérant la restructuration des tissus conjonctifs. Il permet d'aboutir à une régénération complète de sorte que l'apparition de situations pathologiques de type fibrotique ou inflammatoire est évitée. La présence du polysaccharide au sein de la matrice favorise également la pénétration de façon ordonnée après la greffe de la matrice des cellules mésenchymateuses des tissus conjonctifs non minéralisés tels que les fibroblastes, tout en incitant ces mêmes cellules à produire leur propre matrice extracellulaire.

A titre d'exemple, le dispositif médical peut être un pansement.

Selon un mode de réalisation préférée de l'invention les matrices selon l'invention pourront comprendre en outre des cellules mésenchymateuses. émanant de tout tissu conjonctif non minéralisé de façon à constituer un substitut de tissu conjonctif, notamment un substitut de derme, de cartilage ou de tendon. Ce substitut pourra être implanté *in vivo.* La matrice pourra comprendre des cellules mésenchymateuses issues de la moelle ou du sang circulant, des fibroblastes ou des cellules chondrocytaires.

De manière avantageuse, les cellules mésenchymateuses qui coloniseront la matrice, seront des fibroblastes dermiques de façon à constituer un substitut de derme. La matrice pourra également comprendre des kératinocytes de façon à constituer un substitut de peau.
De manière avantageuse, les cellules mésenchymateuses, qui coloniseront la matrice, seront des chondrocytes de façon à constituer un substitut de cartilage.

Selon un autre mode de réalisation, l'invention concerne un support de culture cellulaire caractérisé en ce que la surface du support sur laquelle les cellules sont cultivées comprend le polysaccharide selon l'invention.

Typiquement le polysaccharide est sous forme d'un film, d'une membrane ou d'une structure alvéolée tridimensionnelle ou d'un hydrogel.

Selon un autre mode de réalisation, l'invention concerne un procédé de culture de cellules mésenchymateuses, en particulier de fibroblastes, notamment de fibroblastes dermiques, caractérisé en ce que les dits fibroblastes sont cultivés sur une matrice selon l'invention ou sur un support tel que décrit précédemment.

La présente invention sera mieux illustrée ci-après à l'aide des exemples qui suivent. Ces exemples sont donnés uniquement à titre d'illustration de l'objet de l'invention, dont ils ne constituent en aucune manière une limitation.

### EXEMPLES

### 1 Matériels et Méthodes

### 1.1. Préparation de l'exopolysaccharide HE800 à partir de cultures de Vibrio Diabolicus (la souche HE800).

Des modes de préparation d'HE800 ont été décrits dans les documents suivants : WO 98/38327, Raguénès et al, Int J Syst Bact, 1997, 47, 989-995 et Rougeaux et al, Carbohyd. Res, 1999, 322, 40-45.

### a) Cultures de Vibrio diabolicus

La souche HE800 est cultivée sur milieu 2216E [OPPENHEIMER, J. Mar. Res. 11, 10-18 (1952)] enrichi avec du glucose (30 g/l). La production est effectuée à 30°C et à pH 7,4 en fermenteur de 2 litres contenant 1 litre du milieu 2216E-glucosé. Après 48 heures de culture, le moût présente une faible viscosité (de l'ordre de 40 centipoises à 60 rpm).

### b) Purification de l'exopolysaccharide

Les bactéries sont séparées du moût par une centrifugation à 20 000 g pendant 2 heures, puis le polysaccharide est précipité à partir du surnageant à l'aide d'éthanol pur, puis on effectue plusieurs lavages éthanol/eau en proportions croissantes d'éthanol, selon le procédé décrit par TALMONT et al. [Food Hydrocolloids 5, 171-172 (1991)] ou VINCENT et al. [Appl. Environ. Microbiol., 60, 4134-4141 (1994)]. Le polysaccharide obtenu est séché à 30°C et conservé à température ambiante. 2,5 g de polysaccharide purifié par litre de culture ont ainsi été obtenus.

### 1.2. Obtention des fibroblastes

Les expériences ont été réalisées sur des fibroblastes d'origine dermique et des fibroblastes d'origine gingivale. Ces deux types de cellules mésenchymateuses adoptent vis-à-vis du HE800 un comportement très similaire, par conséquent les résultats obtenus avec les fibroblastes gingivaux sont extrapolables aux fibroblastes dermiques.

### 1.2.1) Milieux de culture :

Les cultures sont effectuées dans un milieu, dit complet, composé, de Dubelco MEM Glutamax I contenant de 100 U/ml de pénicilline 100 µg/ml de streptomycine et 2µg/ml de fungizone (Gibco BRL Cergy Pontoise France) supplémenté ou non (milieu carencé) en sérum de veau foetal (SVF).

### 1.2.2) Origine des prélèvements tissulaires :

Les biopsies dermiques utilisées sont mises en culture dans les 3 heures qui suivent leur prélèvement par le praticien. Les prélèvements utilisés sont obtenus après circoncision, à partir de prépuces d'enfants cliniquement sains. Les biopsies gingivales sont prélevées sur des patients jeunes (moins de 30 ans) exempt de pathologies. Ces biopsies sont situées au niveau de la gencive attachée de prémolaires extraites pour raisons orthodontiques. Ces gencives sont de plus, déclarées cliniquement saines par le praticien. Ces biopsies sont des bribes tissulaires détachées au cours de l'extraction et qui n'ont demandé aucune modification de l'acte opératoire.

### 1.2.3) Mise en culture :

Les prélèvements dermiques et gingivaux sont rincés deux fois dans un milieu DMEM contenant une concentration d'antibiotique supérieure à la normale (pénicilline 6x, streptomycine 4x, fungizone2×) puis ils sont découpés en très petits explants(≈2mm²). Ces explants sont déposés à la pipette Pasteur stérile ou avec la pointe du scalpel, dans un flacon de culture de 25 cm², coté parenchyme sur le plastique. La boîte est relevée et laissée 15 minutes dans cette position pour que les explants, à sec, adhèrent au plastique.
Les explants ayant adhéré sont recouverts de quelques gouttes de DMEM supplémenté à 20 % en sérum de veau foetal (SVF). La boîte de culture est alors placée pendant une nuit dans un incubateur à 37 °C, sous une atmosphère composée de 5 % de CO₂ et de 95 % d'air. Le lendemain le surnageant est remplacé par du milieu frais comprenant 20% de SVF, il est renouvelé par la suite toutes les semaines. Après trois semaines les fibroblastes ont colonisé tout le fond de la boîte (les kératinocytes présents dans l'explant n'adhèrent pas dans ces conditions de culture), on procède alors au repiquage. Les explants sont retirés à l'aide d'une pince, les cellules sont rincées deux fois au PBS, puis trypsinisées (Trypsine EDTA Gibco). La trypsinisation est alors stoppée par l'ajout de DMEM comprenant 10% de SVF. Les cellules sont comptées au compteur (Coulter) puis réensemencées dans plusieurs boîtes de culture. Elles sont, à ce moment, considérées en premier passage et sont entretenues dans un milieu complet contenant 10 % de SVF. Lorsque les cellules sont à nouveau confluentes un autre passage est effectué suivant la même procédure, et ce jusqu'au début des expérimentations.

### 1.3. Préparation des films et culture des fibroblastes

### 1.3.1) Préparation des films d'HE800 et culture des fibroblastes

Un surfactage est effectué par le dépôt de 200µl d'une solution à 2mg/ml d'HE800 au fond des puits de culture (boîte 24 puits, 2cm²). La boîte de culture est déposée sous hotte de culture sur une platine chauffante réglée à 37°C, pendant au moins 5 heures. Après évaporation, un film d'HE800 se constitue au fond de la boîte. Les fibroblastes gingivaux sont ensemencés à raison de 10000 cellules par puits et cultivés pendant 7 jours. Une numération des cellules est effectuée chaque jour, certains puits sont fixés pour l'étude morphologique et l'immuno-détection de l' α-actine des muscles lisses.

### 1.3.2) Préparation de films de collagène et de films composite collagène-HE800 et culture des fibroblastes :

Le collagène utilisé est un collagène de type I acido-soluble (2mg/ml) obtenu à partir de queue de rat (Institut Jacques Boy, Reims). Le surfactage des boîtes de culture s'effectue par le dépôt de 200µl d'un mélange de collagène (40µg total) et d'HE800 (5, 50, ou 200µg total).
La boîte de culture (boîte 24 puits, ou labtek, 2cm² par puits) est déposée sous hotte de culture sur une platine chauffante réglée à 37°C, pendant au moins 5 heures. Après évaporation, un film de collagène avec ou sans HE800 se constitue au fond de la boîte. Des fibroblastes sont ensemencés sur ces films afin de s'assurer de la biocompatibilité de la nouvelle surface de culture.

### 1.3.3) Caractérisation de la structure des films :

Les films de collagène et les films composites sont fixés à l'éthanol absolu -20°C puis réhydratés pour être colorés au rouge sirius ((coloration de Junquera, spécifique des collagènes). Ainsi, en rouge Sirius, sous lumière transmise tous les collagènes sont colorés, mais seuls les collagènes correctement fibrillés sont capables de dévier la lumière polarisée.

### 1.4. Culture en Lattis (matrice de collagène) : préparation de tissu conjonctif non minéralisé équivalent.

Les lattis sont constitués avec le même collagène I que celui utilisé pour former les films collagéniques. Après neutralisation de la solution acide de collagène (3mg/lattis), le gel contenant les cellules et qui est en cours de polymérisation est versé dans une boîte de pétri de 5cm de diamètre. HE800 est ajouté au collagène avant l'addition des cellules à raison de 150µg, 300µg ou 600µg par lattis (respectivement 5%, 10%, et 20% de la quantité totale de collagène)

### 1.4.1) Préparation de la solution stock :

| Composants | Quantité |
|---|---|
| DMEM (poudre) | 5g |
| NaHCO₃ | 1,1g |
| AA non essentiels (100×) | 5 ml |
| H₂O biΔ (stérile) | 17 ml |
| Filtrer | |
| H₂O biΔ (stérile) | 250 ml |

Pour 50 ml de solution stock ajouter 10ml de Sérum de Veau Foetal

### 1.4.2) Préparation des lattis :

Toutes les étapes de préparation du lattis se font dans la glace.

| | |
|---|---|
| solution stock (SVF) | 2,75 ml |
| Collagène (2mg/ml) | 1,5 ml |
| NaOH (0,1N) | 0,25 ml |
| cellules (300000/ml) | 0,5 ml |

- Remuer puis verser dans la boîte de Pétri puis laisser pendant 5 min à 37 °C le lattis.
- Après 1h les boîtes sont légèrement remuées afin de décoller les lattis des rebords.
- Les milieux de culture sont changés chaque semaine.

### 1.4.3) Caractérisation des lattis (des matrices de collagène) :

A différents temps de culture (11 et 40 jours) les lattis sont récupérés fixés dans du paraformaldéhyde puis préparés en vue de l'inclusion en paraffine. Des coupes de 7µm d'épaisseur sont alors effectuées au microtome. Les colorations spécifiques de ces coupes permettent d'observer et d'étudier la structure, la cellularité des tissus conjonctifs reconstruits. Certains des paramètres mis en évidence peuvent par la suite être étudiés par analyse d'images et ainsi être quantifiés. La qualité de la fibrillation collagénique est observée après coloration au rouge Sirius, la cellularité des tissus conjonctifs équivalents a pu être estimée par analyse d'images après coloration des coupes à l'hémalun-éosine.

### 1.4.4) Détermination du nombre de fibroblastes contenus dans les lattis :

La coloration par l'hémalun-éosine permet de distinguer les cellules de la matrice qui les environne. En effet l'hémalun colore les noyaux des cellules en bleu noir, tandis que l'éosine colore en rouge plus ou moins intense les cytoplasmes et les structures extracellulaires (éosinophile). Le contraste ainsi créé permet de distinguer chaque cellule sous un microscope équipé d'une caméra CDD reliée à un analyseur d'images semi-automatique. Les cellules se trouvant dans les champs définis par le grandissement microscopique sont alors comptées dans les lattis à 11 et 40 jours. Une dizaine de champs par coupe a été analysée. Deux groupes de cellules peuvent ainsi être différenciés selon leur situation géographique : les cellules se trouvant à l'intérieur du lattis (matrice de collagène) d'une part et les cellules se trouvant à la périphérie du lattis d'autre part. Pour calculer la cellularité des tissus conjonctifs équivalents chaque lattis est considéré cylindrique, la périphérie du lattis se définissant comme une couronne de 10µm d'épaisseur (équivalente au diamètre de deux assises cellulaires) représentant 2% du volume total du lattis.

### 1.5. Immuno-détection indirecte de l'α-actine des muscles lisses.

Les cellules fixées sont re-perméabilisées dans l'éthanol à 70% (20min), puis réhydratées dans du PBS (10 min). Les peroxydases endogènes sont bloquées avec une solution méthanol (30%), H₂O₂ (0,3%). Cette opération est suivie d'un rinçage au PBS (2min) puis d'un blocage des sites antigéniques non spécifiques par une solution PBS/lait écrémé 1% (1h). Les cultures sont alors incubées avec un anticorps primaire (IgG de souris) dirigé contre l'α-actine humaine (1/30; 50min) puis rincées au PBS (3 × 10 min). Les cellules sont alors incubées, dans le noir pendant 60 min avec un anticorps anti-IgG de souris biotinylé (1/200), rinçées au PBS (3 × 10 min) puis incubées avec de la streptavidine couplée à la peroxydase(1/200).
Après rinçage (PBS 3x 10 min), la révélation de l'activité peroxydasique avec la 3-3' diaminobenzidine s'effectue dans un tampon Tris/HCl (100mM, pH 7,2-7,4) contenant 0,1% de H₂O₂ (15 min, à l'obscurité). L'activité peroxydasique fait apparaître un matériel fibrillaire brun (correspondant aux microfilaments d'α-actine) dans le cytoplasme des cellules positives.
Les produits utilisés proviennent de la firme DAKO. Les expériences contrôles concernant l'immunodétection de l'α-actine des muscles lisses ont été réalisées en omettant l'anticorps primaire et/ou en utilisant un anticorps secondaire d'une autre espèce animale que celle ayant permis l'obtention de l'anticorps primaire.

### 2. Résultats et Discussion

### 2.1. Prolifération des fibroblastes gingivaux cultivés sur film de HE800

Les surfaces de culture ont été traitées par le HE800, afin de former un film polysaccharidique au fond des boîtes. On observe, lors premiers jours de culture (Jours 2 et 4) que le nombre de cellules ensemencées dans les boîtes enduites par le HE800 est largement inférieur au nombre de celles des boîtes témoins (Tableau I). Au dernier jour de l'expérimentation, par contre on remarque une inversion de ces résultats (Tableaux I et II). Les courbes présentées montrent que les cellules cultivées sur film d'HE800 observent un temps de latence avant l'entrée en phase exponentielle de croissance, plus long que celui exprimé par les cellules cultivées sur plastique. D'autre part, alors que le nombre de cellules des cultures témoins atteint un plateau les jours les plus tardifs de l'expérience (cf. Tableau III), les cultures sur film HE800 continuent à proliférer. Les observations effectuées lors de la culture ou après fixation des cellules permettent d'avancer des hypothèses quant aux comportements cellulaires exprimés dans les différentes conditions de culture.

Les cultures sur film d'HE800 se caractérisent lors des premiers jours de cultures par la présence de nombreuses cellules qui n'adhérent pas au support. Cette non-adhésion peut expliquer le retard à la prolifération observée lors des comptages cellulaires dans ces cultures.
Les cellules témoins sont réparties de manière uniforme dans la boîte, sans orientation particulière, alors que les cellules ensemencées sur film HE800 s'organisent en cordons au centre de la boîte. Ces résultats montrent l'incidence d'HE800 sur l'adhésion cellulaire. En effet les regroupements cellulaires qui sont observés habituellement, dans les cultures gingivales, n'ont pas d'orientation spécifique. Après les 2 premiers jours de culture ces cordons cellulaires commencent à former une structure centrale circulaire, allant en se densifiant exclusivement vers le centre (prolifération centripète). On peut aussi observer de nombreuses cellules à la périphérie de la boîte mais sans orientation particulière. Certaines cellules peuvent être présentes dans les zones séparant les regroupements cellulaires, elles sont isolées et semblent beaucoup plus étirées en longueur que les autres cellules des boîtes HE800 ou même témoin. Le marquage immuno-cytochimique concernant l'α-actine des muscles lisses montre :
- Dans les témoins, de nombreuses cellules positives côtoient des cellules n'exprimant pas ces microfilaments.
- Dans les cultures en présence d'HE800, les cellules présentes dans les formations circulaires centrales n'expriment pas l'α-actine des muscles lisses par contre, des cellules exprimant cette isoforme de l'actine peuvent être trouvées en périphérie de la boîte.
Ces résultats sont le reflet d'une sélection des souches fibroblastiques, en effet certaines cellules peuvent ne pas exprimer naturellement les récepteurs membranaires nécessaires à leur adhésion sur le film d'HE800. Parmi les sous-populations fibroblastiques non adhérentes, on retrouve celles qui expriment l'α-actine des muscles lisses c'est-à-dire les myofibroblastes. Dans les expériences contrôles (omission de l'anticorps primaire ou utilisation d'un anticorps secondaire inapproprié) aucune positivité n'à été observée.

**Tableau I : Variation du nombre de cellules par puits au cours de la culture**

| | Jour 2 | Jour 4 | jour 7 |
|---|---|---|---|
| Témoin | 14812 | 45610 | 52980 |
| HE800 | 10035 | 36995 | 64247 |

**Tableau II: Pourcentages de prolifération Tableau III : Temps de doublement (heures)**

| | Jour 2 | Jour 4 | Jour 7 | | | Td 0-2 | Td 2-4 | Td 4-7 |
|---|---|---|---|---|---|---|---|---|
| Témoin | 100 | 100 | 100 | | Témoin | 84, 68 | 29.58 | 333.2 |
| HE800 | 67, 75 | 81, 11 | 127, 27 | | HE800 | 9522, 64 | 25.50 | 90 |

### 2.2. Structuration du collagène de type I en présence d'HE800

### 2.2.1) Premières constatations

Afin de réaliser les films comprenant à la fois du collagène et de l'exopolysaccharide HE800, des solutions d'HE800 et de collagène I sont préalablement mélangées avant dépôt sur les boîtes de culture. De manière surprenante, il a été constaté que l'addition de l'exopolysacharide bactérien à la solution collagénique provoque l'apparition d'un agglomérat dense de couleur blanche. Cet agglomérat a pu être étalé sur une lame histologique puis colorée au rouge Sirius, colorant spécifique des collagènes. Ces lames histologiques montrent qu'effectivement le matériel étalé sur la lame est coloré par le rouge Sirius. De plus l'observation d'un matériel déviant dans différentes directions la lumière polarisée montre bien la présence de collagène sous sa forme fibrillaire.

### 2.2.2) Organisation de films composites collagène/HE800:

Les différents films déposés sont composés de :
- (1) collagène (40µg)
- (2) collagène (40µg) + HE800 (50µg)
- (3) collagène (40µg) + HE800 (200µg)

L'observation au microscope du fond des boîtes montre pour les films (3) l'apparition d'un réseau dense composé de longs filaments. Les films (2) comportent quelques fibres beaucoup plus courtes, tandis que les films (1) n'en comportent pratiquement pas. Les 3 films se colorent au rouge Sirius, mais seul le film (3) montre un réseau fibrillaire déviant la lumière polarisée.
Ces résultats indiquent que HE800 promeut la formation de fibres collagéniques, mais aussi permet une meilleure résistance de la trame collagénique devant des facteurs physiques tels que la température et les contraintes mécaniques.

### 2.3. Tissu conjonctif non minéralisé: Microscopie photonique et électronique

Les cellules sont cultivées dans une matrice collagénique (modèle de culture tridimensionnelle) afin de mimer au mieux les interactions cellules/matrice observées dans le tissu conjonctif. Ces lattis ou tissus conjonctifs équivalents sont composés de collagène I seul (Témoins) ou de collagène I et d'HE800 en différentes proportions (quantité d'EPS = 20, 10 et 5% par rapport à la quantité de collagène contenu dans le lattis, c'est-à-dire respectivement 300, 150 et 75µg).

### 2.3.1) Rétraction des lattis :

Le premier paramètre étudié est la vitesse de rétraction des lattis : Les courbes de rétraction des lattis témoins et des lattis comprenant de l'HE800 sont similaires. Malgré ces similarités, on remarque que les lattis HE800 ont une vitesse de rétraction plus lente que les lattis témoins lors des jours précoces de culture. Après le 11^{ème} jour la rétraction des lattis est presque totale.

### 2.3.2) Nombre de fibroblastes contenus dans les lattis :

Le nombre de cellules présentes dans chaque lattis varie, aux deux temps de culture entre 180 000 et 250 000 cellules. Le nombre de cellules à la périphérie représente 2 à 12% du nombre total de cellules. Ces données sont compatibles avec ce qui a été décrit dans la littérature pour ce modèle de culture. Les résultats des tableaux IV, V et VI montrent le nombre de cellules par unité volumétrique (mm³) présentes dans la totalité du lattis et dans ses différentes régions. Les densités cellulaires volumétriques totales des lattis après 11 et 40 jours sont comprises entre 3200 et 5900 cellules/mm³ (cf. tableau IV). Ces valeurs sont comparables à celles retrouvées dans un tissu conjonctif humain normal comme cela à été décrit précédemment (Miller et al., Exp Dermatol. 2003 Aug;12(4):403-11). La cellularité physiologique des lattis témoins et des lattis comprenant du HE800 atteste donc de la validité du modèle de culture utilisé et de la compatibilité d'HE800 avec ce modèle physiologique.
La densité cellulaire totale (cf. tableau IV) des lattis témoins ne varie pas quelque soit le temps de culture. Au 11^{ème} jour de culture, la densité cellulaire totale des lattis HE800 est inférieure de 25 à 40% à celles des lattis témoin. Au 40^{ème} jour de culture les densités cellulaires des lattis témoins et des lattis HE800 sont équivalentes. Les variations de densités cellulaires observées à l'intérieur des lattis (cf. tableau V) reproduisent exactement celles de la totalité du lattis. L'organisation topologique des cellules de la couronne périphérique (tableau VI) diverge par contre totalement de celles des tableaux IV et V :
- A 11 jours de culture les densités cellulaires de la couronne périphérique sont 4 fois supérieures à celles de l'intérieur du lattis et ne présentent que peu de variation entre les tissus conjonctifs équivalents témoins et les tissus conjonctifs équivalents comprenant HE800 (tableau VI).
- A 40 jours de culture, une forte diminution de la cellularité périphérique est observée. Si cette diminution n'est que de 40% pour les lattis témoins, elle atteint 100 à 250% pour les lattis contenant du HE800. La densité cellulaire des lattis témoin reste 3 fois plus élevée à la périphérie qu'à l'intérieur (tableau IV et VI) par contre ces densités sont comparables pour les lattis HE800.
La cellularité globale des lattis HE800 est plus faible que celle des lattis témoins aux jours précoces de culture puis deviennent équivalentes aux jours tardifs de culture. Ces variations qui se répercutent sur les densités cellulaires des régions internes peuvent s'expliquer par une stimulation de la prolifération cellulaire, ou une migration massive de cellules périphériques vers l'intérieur.
En effet à la périphérie des lattis, le nombre de cellules diminue au cours du temps de culture, cette diminution est particulièrement accentuée dans les lattis comprenant du HE800 (diminution par 2 à 3,5 fois du nombre de cellules). Cette diminution peut s'expliquer par, une perte d'adhésion des cellules périphérique qui se détache de la matrice extracellulaire et/ou une migration massive de ces cellules à l'intérieur. Cela explique les gains globaux de cellularité, au cours du temps, dans les lattis contenant HE800.

**Tableau IV : Densité cellulaire dans la totalité du lattis : nombre de cellule par mm³ (entre parenthèse : diamètre des lattis en mm).**

| Temps de culture | 11 jours | 40 jours | Variation entre 11 et 40 jours |
|---|---|---|---|
| Témoins | 5924 (8,3) | 5695 (8) | - 4% |
| HE800 20% | 4468 (8) | 6150 (8) | + 27% |
| HE800 10% | 3899 (9,7) | 6000 (8) | + 35% |
| HE800 5% | 3491 (1,03) | 5023 (8) | + 30% |

**Tableau V : Densité cellulaire à l'intérieur du lattis : nombre de cellule par mm³.**

| Temps de culture | 11 jours | 40 jours | Variation entre 11 et 40 jours |
|---|---|---|---|
| Témoins | 5568 | 5520 | - 3% |
| HE800 20% | 4153 | 6085 | + 22% |
| HE800 10% | 3517 | 5938 | + 41% |
| HE800 5% | 3220 | 5005 | + 36% |

**Tableau VI: Densité cellulaire à la périphérie du lattis: nombre de cellule par mm³**

| Temps de culture | 11 jours | 40 jours | Variation entre 11 et 40 jours |
|---|---|---|---|
| Témoins | 20134 | 14382 | - 40% |
| HE800 20% | 23367 | 6531 | - 258% |
| HE800 10% | 22161 | 9979 | - 122% |
| HE800 5% | 17731 | 5939 | - 199% |

**Conclusion : HE800 favorise la prolifération des fibroblastes dermiques dans la matrice extracellulaire et/ou favorise leur mobilisation c'est-à-dire la sélection, la migration et la pénétration massive des cellules périphériques.**

### 2.4.3) Etat de la matrice collagénique :

### 2.4.3.1) Microscopie photonique

La coloration au rouge Sirius (coloration de Junquera) permet de colorer spécifiquement les collagènes, dans la peau par exemple ses collagènes apparaissent sous la forme d'une structure filamenteuse lâche, colorée en rouge.

Les colorations au rouge Sirius des coupes histologiques après observation en lumière transmise et lumière polarisée montrent que l'addition d'HE800 lors de la formation du lattis permet la formation d'une matrice beaucoup plus dense et à des temps beaucoup plus courts que dans les lattis témoins.
Pour exemple : la densité de la matrice collagénique témoin après 40 jours de culture est équivalente à celle observée dans les matrices collagéniques constituées en présence de HE800 à 11 jours de culture. Cet effet sur la densité est beaucoup plus important aux doses les plus faibles (10%, 5%).

### 2.4.3.2) Microscopie électronique

La microscopie électronique a été pratiquée sur des tissus conjonctifs équivalents cultivés pendant 11 jours. Un bon état ultrastructural des cellules a été observé, que ce soit dans les témoins ou que ce soit dans les lattis formés en présence des différentes concentrations d'HE800.

Aucunes fibres collagéniques n'a pu être observées dans les lattis témoins, les lattis comprenant 20% de HE800 permettent d'observer quelques éléments fibrillaires pris dans un gel constitué de l'exopolysaccharide. Les lattis comprenant 10% et 5 % d'exopolysaccharides sont très différents, en effets de nombreuses fibres collagéniques sont présentes, elles sont réparties dans tout le lattis et présentent, pour certaines, une striation périodique. Ces fibres ou fibrilles collagéniques sont piégées dans le gel constitué par l'HE800.

**Conclusion : L'HE800 accélère la fibrillation collagénique et favorise la constitution d'une matrice extracellulaire.**

## Revendications

1. Matrice de collagène comprenant un polysaccharide ou un sel de ce polysaccharide de masse molaire moyenne en poids comprise entre 500 000 et 2 000 000 g/mol, préférentiellement comprise entre 700 000 et 900 000 g/mol, **caractérisé par** une séquence osidique répétitive linéaire comprenant les 4 résidus osidiques suivants :
**[(-3)-DGlcNacβ(1-4)DGlcAβ(1-4)DGlcAβ(1-4)DGalNacα(1-)]**

2. Matrice selon la revendication 1 **caractérisée en ce que** ledit polysaccharide est un polysaccharide excrété par l'espèce *Vibrio diabolicus.*

3. Matrice de collagène selon la revendication 1, **caractérisée en ce que** le collagène est un collagène choisi dans le groupe des collagènes fibrillaires constitué des collagènes de type I, II III, V et XI ou d'un mélange de ceux-ci.

4. Matrice selon la revendication 3 **caractérisé en ce que** le collagène est un collagène de type I.

5. Matrice comprenant le polysaccharide tel que défini dans l'une quelconque des revendications 1 à 4 **caractérisé en ce que** le polysaccharide a été rendu insoluble par réticulation à l'aide d'un ou plusieurs agents réticulants.

6. Matrice selon l'une quelconque des revendications 1 à 5 comprenant en outre un ou plusieurs facteurs de croissance qui favorisent la colonisation de la matrice par les cellules mésenchymateuses, en particulier par les fibroblastes.

7. Matrice selon la revendication 6 **caractérisée en ce que** le ou les facteurs de croissance sont choisis dans le groupe consistant en TGF-beta, PDGF, FGF, VEGF, BMPs, CTGF.

8. Matrice selon l'une quelconque des revendications 1 à 7 comprenant en outre des cellules mésenchymateuses.

9. Matrice selon la revendication 8 **caractérisée en ce que** les cellules mésenchymateuses sont des cellules issues de la moelle ou du sang circulant, des fibroblastes ou des cellules du cartilage.

10. Matrice selon la revendication 9 **caractérisée en ce que** les cellules mésenchymateuses sont des fibroblastes dermiques.

11. Matrice selon la revendication 10 comprenant en outre des kératinocytes.

12. Matrice selon la revendication 8 ou 9 **caractérisée en ce que** les cellules mésenchymateuses sont des chondrocytes.

13. Substitut de tissu conjonctif non minéralisé comprenant la matrice selon l'une quelconque des revendications 8 à 12.

14. Substitut selon la revendication 13 **caractérisé en ce que** le substitut est un substitut de tendon.

15. Substitut de derme comprenant la matrice selon la revendication 10.

16. Substitut de peau comprenant la matrice selon la revendication 11.

17. Substitut de cartilage comprenant la matrice selon la revendication 12.

18. Dispositif médical ou implant comprenant la matrice selon l'une quelconque des revendications 1 à 7.

19. Dispositif médical selon la revendication 18 **caractérisé en ce que** le dispositif médical est un pansement.

20. Procédé de culture in vitro de cellules mésenchymateuses, en particulier de fibroblastes, **caractérisé en ce que** les cellules mésenchymateuses sont cultivées sur la matrice selon l'une quelconque des revendications 1 à 7.

21. Utilisation in vitro d'une matrice selon l'une des revendications 1 à 12 pour la fabrication d'un substitut de tissu conjonctif non minéralisé ou d'un implant utile en ingénierie tissulaire.

## Patentansprüche

1. Kollagenmatrix, umfassend ein Polysaccharid oder ein Salz des Polysaccharids mit einer mittleren molaren Masse, bezogen auf das Gewicht, umfassend zwischen 500000 und 2000000 g/mol, vorzugsweise umfassend zwischen 700000 und 900000 g/mol, **gekennzeichnet durch** eine repetitive lineare Osid-Sequenz, umfassend die 4 folgenden Osid-Reste: **[(-3)-DGlcNacβ(1-4)DGlcAβ(1-4)DGlcAβ(1-4)DGalNacα(1-)]**

2. Matrix nach Anspruch 1, **dadurch gekennzeichnet, dass** das Polysaccharid ein Polysaccharid ist, das durch die Spezies Vibrio diabolcus sekretiert wird.

3. Kollagenmatrix nach Anspruch 1, **dadurch gekennzeichnet, dass** das Kollagen ein Kollagen ist, ausgewählt aus der Gruppe der fibrillären Kollagene, bestehend aus den Kollagenen des Typs I, II, III, V und XI oder einem Gemisch dieser.

4. Matrix nach Anspruch 3, **dadurch gekennzeichnet, dass** das Kollagen ein Kollagen des Typs I ist.

5. Matrix, umfassend das Polysaccharid wie in einem der Ansprüche 1 bis 4 definiert, **dadurch gekennzeichnet, dass** das Polysaccharid unlöslich gemacht wurde durch Vernetzung mit Hilfe eines oder mehrerer Vernetzungsmittel.

6. Matrix nach einem der Ansprüche 1 bis 5, weiterhin umfassend einen oder mehrere Wachstumsfaktoren, die die Besiedelung der Matrix durch die mesenchymalen Zellen, insbesondere durch die Fibroblasten, begünstigen.

7. Matrix nach Anspruch 6, **dadurch gekennzeichnet, dass** der Wachstumsfaktor oder die Wachstumsfaktoren ausgewählt sind aus der Gruppe, bestehend aus TGF-beta, PDGF, FGF, VEGF, BMPs, CTGF.

8. Matrix nach einem der Ansprüche 1 bis 7, weiterhin umfassend mesenchymale Zellen.

9. Matrix nach Anspruch 8, **dadurch gekennzeichnet, dass** die mesenchymalen Zellen Zellen aus dem Rückenmark oder Kreislaufblut, Fibroblasten oder Knorpelzellen sind.

10. Matrix nach Anspruch 9, **dadurch gekennzeichnet, dass** die mesenchymalen Zellen dermale Fibroblasten sind.

11. Matrix nach Anspruch 10, weiterhin umfassend Keratinozyten.

12. Matrix nach Anspruch 8 oder 9, **dadurch gekennzeichnet, dass** die mesenchymalen Zellen Chondrozyten sind.

13. Nichtmineralisiertes Bindegewebeersatzmaterial, umfassend die Matrix nach einem der Ansprüche 8 bis 12.

14. Ersatzmaterial nach Anspruch 13, **dadurch gekennzeichnet, dass** das Ersatzmaterial ein Sehnenersatzmaterial ist.

15. Dermisersatzmaterial, umfassend die Matrix nach Anspruch 10.

16. Hautersatzmaterial, umfassend die Matrix nach Anspruch 11.

17. Knorpelersatzmaterial, umfassend die Matrix nach Anspruch 12.

18. Medizinprodukt oder Implantat, umfassend die Matrix nach einem der Ansprüche 1 bis 7.

19. Medizinprodukt nach Anspruch 18, **dadurch gekennzeichnet, dass** das Medizinprodukt ein Verband ist.

20. Verfahren zum in vitro-Kultivieren von mesenchymalen Zellen, insbesondere Fibroblasten, **dadurch gekennzeichnet, dass** die mesenchymalen Zellen kultiviert werden auf der Matrix nach einem der Ansprüche 1 bis 7.

21. In vitro-Verwendung einer Matrix nach einem der Ansprüche 1 bis 12 zur Herstellung eines nichtmineralisierten Gewebeersatzmaterials oder eines Implantats, das in der Gewebetechnik verwendbar ist.

## Claims

1. Collagen matrix comprising a polysaccharide or a salt of this polysaccharide having a weight-average molar mass of between 500 000 and 2 000 000 g/mol, preferably between 700 000 and 900 000 g/mol, **characterized by** a linear repeating oside sequence comprising the following 4 oside residues:
**[(-3)-DGlcNacβ(1-4)DGlcAβ(1-4)DGlcAβ(1-4)DGalNacα(1-)]**

2. Matrix according to Claim 1, **characterized in that** said polysaccharide is a polysaccharide excreted by the *Vibrio diabolicus* species.

3. Collagen matrix according to Claim 1, **characterized in that** the collagen is a collagen chosen from the group of fibrillar collagens consisting of collagen type I, II, III, V and XI or of a mixture thereof.

4. Matrix according to Claim 3, **characterized in that** the collagen is a type I collagen.

5. Matrix comprising the polysaccharide as defined in any one of Claims 1 to 4, **characterized in that** the polysaccharide has been rendered insoluble by crosslinking using one or more crosslinking agents.

6. Matrix according to any one of Claims 1 to 5, further comprising one or more growth factors which promote colonization of the matrix by mesenchymal cells, in particular by fibroblasts.

7. Matrix according to Claim 6, **characterized in that** the growth factor (s) is (are) chosen from the group consisting of TGF-beta, PDGF, FGF, VEGF, BMPs and CTGF.

8. Matrix according to any one of Claims 1 to 7, further comprising mesenchymal cells.

9. Matrix according to Claim 8, **characterized in that** the mesenchymal cells are cells derived from the marrow or from circulating blood, fibroblasts or cartilage cells.

10. Matrix according to Claim 9, **characterized in that** the mesenchymal cells are dermal fibroblasts.

11. Matrix according to Claim 10, further comprising keratinocytes.

12. Matrix according to Claim 8 or 9, **characterized in that** the mesenchymal cells are chondrocytes.

13. Non-mineralized connective tissue substitute comprising the matrix according to any one of Claims 8 to 12.

14. Substitute according to Claim 13, **characterized in that** the substitute is a tendon substitute.

15. Dermal substitute comprising the matrix according to Claim 10.

16. Skin substitute comprising the matrix according to Claim 11.

17. Cartilage substitute comprising the matrix according to Claim 12.

18. Medical device or implant comprising the matrix according to any one of Claims 1 to 7.

19. Medical device according to Claim 18, **characterized in that** the medical device is a dressing.

20. Method for the *in vitro* culture of mesenchymal cells, in particular of fibroblasts, **characterized in that** the mesenchymal cells are cultured on the matrix according to any one of Claims 1 to 7.

21. *In vitro* use of a matrix according to one of Claims 1 to 12, for the production of a non-mineralized connective tissue substitute or of an implant that can be used in tissue engineering.
